# EUROPEAN PATENT SPECIFICATION

(11) **EP 4 705 718 B1**
(45) Date of publication and mention of the grant of the patent: **04.11.2026**
(21) Application number: 24716238.1
(22) Date of filing: 28.03.2024
(51) Int. Cl.: G01B 9/02091, G01B 9/02004, G01B 9/02, G06F 17/16

(54) **METHOD AND SYSTEM FOR OPTICAL COHERENCE TOMOGRAPHY EXPLOITING TRUNCATED SINGULAR VALUE DECOMPOSITION**
VERFAHREN UND SYSTEM FÜR OPTISCHE KOHÄRENZTOMOGRAFIE UNTER AUSNUTZUNG VON VERKÜRZTER EINZELWERTZERLEGUNG
PROCÉDÉ ET SYSTÈME DE TOMOGRAPHIE PAR COHÉRENCE OPTIQUE EXPLOITANT UNE DÉCOMPOSITION EN VALEURS SINGULIÈRES TRONQUÉES

(30) Priority: 03.05.2023 IT 202300008748
(43) Date of publication of application: 11.03.2026
(73) Proprietor: Careglance Srl, 20135 Milano (IT)
(72) Inventor: ACHINI, Federico, 20135 Milano (IT); IANNONE, Eugenio, 20135 Milano (IT)
(74) Representative: Tirloni, Bartolomeo
(86) International application number: PCT/IB2024/053036
(87) International publication number: WO 2024/228068

(56) References cited:
- BADON AMAURY ET AL: "Smart optical coherence tomography for ultra-deep imaging through highly scattering media", vol. 2:e1600370, 1 November 2016 (2016-11-01), Washington, D.C., pages 1 - 9, XP093093774, ISBN: 978-1-943580-29-3, Retrieved from the Internet <URL:https://www.science.org/doi/pdf/10.1126/sciadv.1600370> DOI: 10.1364/MATH.2017.MW3C.1
- RALSTON T S MARKS D L ET AL: "Deconvolution Methods for Mitigation of Transverse Blurring in Optical Coherence Tomography", IEEE TRANSACTIONS ON IMAGE PROCESSING, IEEE, USA, vol. 14, no. 9, 1 September 2005 (2005-09-01), pages 1254 - 1264, XP002472451, ISSN: 1057-7149, DOI: 10.1109/TIP.2005.852469
- YANG LU ET AL: "Optimized number of the primary singular values for image reconstruction in reflection matrix based optical coherence tomography", vol. 30, no. 2, 12 January 2022 (2022-01-12), pages 2680, XP093093735, Retrieved from the Internet <URL:https://opg.optica.org/directpdfaccess/98f37395-d926-46a9-9de06bb5ca1e4173_468430/oe-30-2-2680.pdf?da=1&id=468430&seq=0&mobile=no> DOI: 10.1364/OE.442672

## Description

### Background art

### Technical field

The present disclosure generally relates to an Optical Coherence Tomography (OCT) system, such as a "Swept Source - Optical Coherence Tomography" (SS-OCT) system. More particularly, the present disclosure relates to a SS-OCT system adapted to be used in ophthalmologic applications.

### Overview of the related art

A SS-OCT system uses a coherent light signal to capture two and three dimensional images of a sample, hereinafter referred to as "target object" or more generally as "object". SS-OCT is particularly used in those applications in which a non-destructive testing of the object is needed. The object may be any object comprising different kinds of materials, such as biological tissue.

A conventional SS-OCT system comprises a light source (such as a wavelength-swept laser source) configured to generate the coherent light signal.

A conventional SS-OCT system also comprises an optical interferometer. According to a typical configuration, the optical interferometer comprises a reference arm leading to a reference reflector (for example, a reference mirror), an operative arm leading to the object, and a beam splitter configured to split the coherent light signal into a reference forward signal directed to the reference arm, and into an operative forward signal directed to the object arm. Other optical interferometer configurations may also be envisaged.

A conventional SS-OCT system further comprises an optical detector (such as an optical homodyne receiver, particularly a balanced optical homodyne receiver) configured to detect an interference signal generated by a combination of a reference return signal returning from the reference arm after reflection (of the reference forward signal) by the reference reflector, and an operative return signal returning from the operative arm after reflection or backscattering (of the sample forward signal) by the object.

A processing unit is configured to perform an image reconstruction or synthesis procedure (hereinafter concisely referred to as reconstruction procedure) aimed at providing, based on the detected interference signal, one or more images of the object.

An optical scanning element (referred to as optical scanner) is typically provided (e.g., between an end of the operative arm and the object) to scan the operative forward signal on the object. According to a typical operation mode, the optical scanner is configured to direct the operative forward signal to a target point in a resting plane where the object at least partially lies, so as to obtain an axial scan (A-scan) based on a profile of the corresponding operative return signal as a function of a direction (or depth) orthogonal to the resting plane; after the end of an A-scan, the optical scanner is moved to a different target point in the resting plane, so as to perform another A-scan.

A set of A-scans associated with neighboring target points in the object may be used, by the reconstruction procedure, to generate a cross-sectional image of the object (referred to as B-scan), and a set of B-scans through the resting plane may be used by the reconstruction procedure to generate a tridimensional (3D) image of the object (referred to as C-scan).

The reconstruction procedure is essentially based on inverting (and solving) the equation providing the detected interference signal as a function of the reflectivity of the object.

The most common approach for solving the equation of the detected interference signal is based on the Fourier transform of the detected interference signal.

An alternative well known approach provides for arranging the equation of the detected interference in a Fredholm form, with a kernel depending on time and space, and solving the Fredholm equation through a time domain algorithm for obtaining the reflectivity.

Known time domain algorithms for solving Fredholm equation provide for discretizing the equation in time - the interference signal is sampled at corresponding sample times - and space -the reflectivity is calculated at corresponding space samples within the object - to depict the kernel in matrix form ("kernel matrix"), applying an eigenvalue decomposition to the kernel matrix, and obtain for each space sample a reflectivity value depending on the eigenvalues of the kernel matrix. This known procedure applies to cases in which the number of time samples is equal to the number of space samples, so that the kernel matrix is a square matrix. Since the number of time samples is usually much larger than the number of space samples, the kernel matrix is usually a rectangular matrix. In this case, the eigenvalue decomposition is substituted (generalized) by the Singular Values Decomposition (SVD), and the obtained reflectivity values depend on the singular values of the kernel matrix.

Paper "Smart optical coherence tomography for ultra-deep imaging through highly scattering media" by Amaury Badon, Dayan Li, Geoffroy Lerosey, A. Claude Boccara, Mathias Fink and Alexandre Aubry, Imaging and Applied Optics 2017 (3D, AlO, COSI, IS, MATH, pcAOP), vol. 2, 1 November 2016, pages 1-9, proposes a matrix approach of optical imaging. Paper by RALSTON T S MARKS D L ET AL: "Deconvolution Methods for Mitigation of Transverse Blurring in Optical Coherence Tomography", IEEE TRANSACTIONS ON IMAGE PROCESSING, IEEE, USA, vol. 14, no. 9, 1 September 2005 (2005-09-01), pages 1254-1264, discloses a system and method for Optical Coherence Tomography.

### Summary

Solving the equation of the detected interference signal using the known methods described above is affected by drawbacks capable of invalidating the accuracy of the results.

More particularly, two problems are present. First, using the known approaches the singular values of the kernel matrix are obtained by sampling sinusoidal functions whose phase depends on the absolute value of the optical frequency of the coherent light signal. Since the optical frequency of the coherent light signal is substantially large, phase errors are greatly amplified, so that even a small error in the phase is capable of considerably disrupting the accuracy of the final results.

Moreover, when solving the equation of the detected interference signal using the known time domain algorithms, the solutions are in a form comprising fractions having a numerator corresponding to the detected interference signal and a denominator corresponding to the singular values of the kernel matrix. Since the numeric algorithms used by the known method described above imply the presence of unavoidable numerical errors affecting the detected interference signal, such numerical errors can be greatly amplified by the presence of small singular values of the kernel matrix that are close to zero.

In view of the above, the Applicant has devised an OCT system capable of overcoming the above-mentioned, as well as other issues.

One or more aspects of the present disclosure are set out in the independent claims, with advantageous features of the same disclosure that are indicated in the dependent claims, whose wording is enclosed herein verbatim by reference (with any advantageous feature being provided with reference to a specific aspect of the present disclosure that applies mutatis mutandis to any other aspect thereof).

An aspect of the present invention relates to an Optical Coherence Tomography (OCT) system according to claim 1.

According to an embodiment of the present invention, each weight corresponds to the inverse of a respective one of said singular values.

According to an embodiment of the present invention, said coefficient matrix depends on at least one among:
- refraction index of the target object;
- the sampling times;
- the predetermined depths;
- a rate of variation over time of the wavelength of coherent light signal.

According to an embodiment of the present invention, each singular value corresponding to a respective electrical signal sample, apart from the singular value corresponding to the first electrical signal sample of the sequence, is not higher than the singular value corresponding to the previous electrical signal sample in the sequence.

According to an embodiment of the present invention, the processing unit is configured to select said subset of the received electrical signal samples by selecting electrical signal samples corresponding to singular values higher than a threshold. According to an embodiment of the present invention, said threshold is equal to a fraction of the highest singular value among the singular values obtainable by the decomposition of said coefficient matrix.

According to an embodiment of the present invention, the processing unit is configured to select said subset of the received electrical signal samples by selecting, among all the electrical signal samples of the sequence, a subsequence comprising initial electrical signal samples in the sequence and by discarding subsequent electrical signal samples in the sequence. According to an embodiment of the present invention, said subset of the received electrical signal samples comprises a number of the received electrical signal samples, said number depending on the difference between the highest and lower wavelength of the coherent light signal during said time interval.

According to an embodiment of the present invention, the unknown variables of said system of linear equations comprise the reflectivity values of the set multiplied by a term depending on phase values of said electrical signal samples, said phase values being functions of said predetermined depths.

According to an embodiment of the present invention, the processing unit is configured to generate an A-scan of the target object based on the set of the reflectivity values.

According to an embodiment of the present invention the target object is a retinal zone of an eye.

According to an embodiment of the present invention, the wavelength of the coherent light spans, during said time interval, a wavelength sweep interval comprising a wavelength corresponding to 1064 nm.

According to an embodiment of the present invention, the difference between the highest and lower wavelength of the coherent light in said wavelength sweep interval corresponds to 11 nm, and the processing unit is configured to select said subset of the received electrical signal samples by selecting current samples corresponding to singular values higher than a threshold lower than 90, preferably corresponding to 40.

According to an embodiment of the present invention, the difference between the highest and lower wavelength of the coherent light in said wavelength sweep interval corresponds to 22 nm, and the processing unit is configured to select said subset of the received electrical signal samples by selecting current samples corresponding to singular values higher than a threshold lower than 65, preferably corresponding to 20.

According to an embodiment of the present invention, the difference between the highest and lower wavelength of the coherent light in said wavelength sweep interval corresponds to 44 nm, and the processing unit is configured to select said subset of the received electrical signal samples by selecting current samples corresponding to singular values higher than a threshold lower than 45, preferably corresponding to 5.

According to an embodiment of the present invention, the difference between the highest and lower wavelength of the coherent light in said wavelength sweep interval corresponds to 110 nm, and the processing unit is configured to select said subset of the received electrical signal samples by selecting current samples corresponding to singular values higher than a threshold lower than 40, preferably corresponding to 31.

Another aspect of the present invention provides for a method for carrying out an Optical Coherence Tomography (OCT) on a target object according to claim 12.

### Brief description of the drawings

These and other features and advantages of the disclosure will be made apparent by the following description of some exemplary and non-limitative embodiments thereof. For its better intelligibility, the following description should be read making reference to the attached drawings, wherein:
**Figure 1** schematically shows a system according to embodiments of the present invention;
**Figures 2A-2D** are diagrams showing four examples of sets of singular values.
**Figure 3A** is a flowchart illustrating in terms of functional blocks the operations performed by a processing unit of the system of **Figure 1** according to an embodiment of the present invention;
**Figure 3B** illustrates in terms of functional blocks modules of the processing unit of the system of **Figure 1** according to an embodiment of the present invention;
**Figures 4A** and **4B** are diagram illustrating exemplary performances of the system according to embodiments of the present invention.

### Detailed description of exemplary embodiments

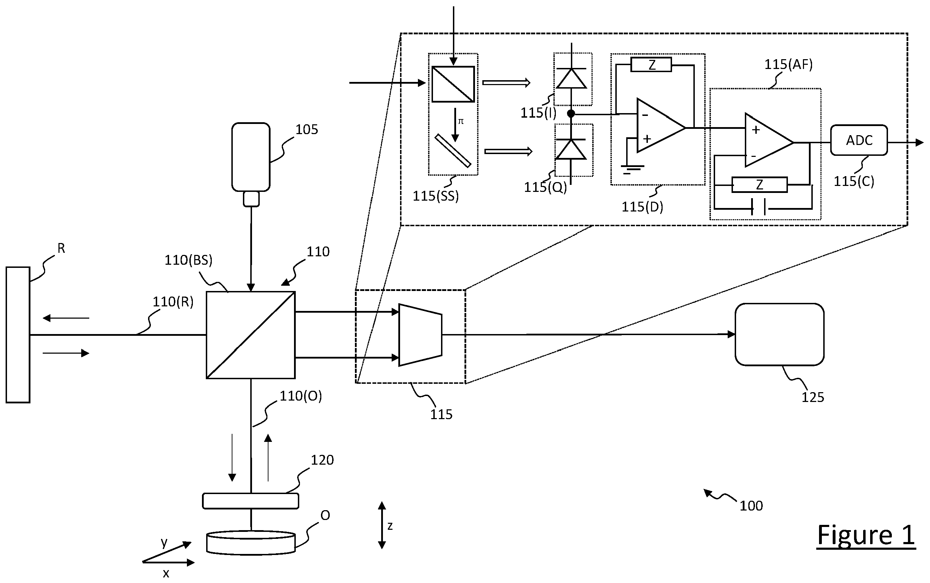

With reference to the drawings, **Figure** 1 schematically shows a system **100** according to the present invention. In the example here considered, the system **100** is an "Optical Coherence Tomography" (OCT) system, particularly a "Swept Source - Optical Coherence Tomography" (SS-OCT) system.

According to an embodiment of the present invention, the system **100** comprises a light source **105** configured to generate a coherent light signal (e.g., a light beam), such as an infrared light signal.

According to an embodiment of the present invention, the light source **105** comprises a laser source. According to the invention, in which the system **100** comprises a SS-OCT system, the light source **105** may comprise a wavelength-swept laser source.

According to the present invention, the system **100** comprises an optical interferometer **110.**

According to the present invention, the optical interferometer **110** comprises a reference arm **110(R)** leading to a reference reflector **R** (the reference reflector **R** being for example at an end of the reference arm **110(R)).**

According to an embodiment of the present invention, the reference reflector **R** comprises a reference mirror.

According to the present invention, the optical interferometer **110** comprises on operative arm **110(0)** leading to a target object **O** (the object **O** being for example at an end of the operative arm **110(0)).**

According to an embodiment of the present invention, the reference arm **110(R)** and/or the operative arm **110(0)** may comprise optical fibers.

Without losing generality, the object **O** may comprise biological tissue, such as the retinal zone of a human eye.

According to an embodiment of the present invention, the optical interferometer **110** comprises a beam splitter **110(BS).** According to an embodiment of the present invention, the beam splitter **110(BS)** is configured to split the coherent light signal (from the light source **105)** into a reference forward signal directed to the reference arm **110(R),** and into an operative forward signal directed to the operative arm **110(0).**

According to the present invention, the system **100** comprises an optical detector **115** configured tc detect an interference signal generated by a combination of a reference return signal returning from the reference arm **110(R)** after reflection (of the reference forward signal) by the reference reflector **R,** and an operative return signal returning from the operative arm **110(0)** after reflection or backscattering (of the operative forward signal) by layers of the object **O**. According to an embodiment of the present invention, the optical detector **115** comprises an optical homodyne receiver, particularly a balanced optical homodyne receiver.

An exemplary embodiment of the optical detector **115** is schematically illustrated in the enlarged view of **Figure 1****.**

According to an embodiment of the present invention, the optical detector **115** comprises a splitting/shifting arrangement **115(SS)** for splitting and phase shifting the interference signal (from the reference **110(R)** and operative **110(O)** arms) into first and second interference signals.

According to an embodiment of the present invention, the optical detector **115** comprises a first photodiode **115(I)** configured to receive the first interference signal, and to generate a corresponding detection current.

According to an embodiment of the present invention, the optical detector **115** comprises a second photodiode **115(Q)** configured to receive the second interference signal, and to generate a corresponding detection current.

According to an embodiment of the present invention, the first and second photodiodes (hereinafter, also referred to as photodiodes **115(Q), 115(I)**) are identical photodiodes.

According to an embodiment of the present invention, the photodiodes **115(Q), 115(I)** have opposite electrical polarizations.

According to an embodiment of the present invention, the optical detector **115** comprises a transimpedance amplifier **115(D)** configured to subtract the detection currents to each other to obtaining a corresponding detected interference current signal, and to convert preferably with an amplification factor the detected interference current signal into a corresponding voltage signal.

According to an embodiment of the present invention, the optical detector **115** comprises an amplification and filtering stage **115(AF)** (for example, a narrow-band, high gain amplifier) for amplifying the voltage signal, and for filtering it from noise and/or disturbances due to phase shifting at the splitting/shifting arrangement **115(SS)** and/or to subtraction operation at the transimpedance amplifier **115(D).**

According to an embodiment of the present invention, the optical detector **115** comprises an analog to digital converter **115(C)** configured to convert (by sampling and quantization operations) the (analog) amplified voltage signal into a corresponding sequence of digital samples (representing the detected interference current signal) adapted to be processed by a processing unit (discussed here below). The sequence of digital samples is a digital and processed version of the detected interference current signal.

According to an embodiment of the present invention, the system **100** comprises (*e.g.*, between the end of the operative arm **110(0)** and the object **O**) an optical scanning element (referred to as optical scanner) **120** configured to scan the operative forward signal on the object **O**.

According to an embodiment of the present invention, the optical scanner **120** is configured to direct the operative forward signal to a target point in a **x-y** plane where the object **O** at least partially lies.

According to the present invention, the system **100** comprises a processing unit **125** configured tc calculate the reflectivity of the object **O** as a function of a **z** direction (or depth), orthogonal to the **x-y** plane, based on the detected interference current signal, so as to generate a corresponding axial scan (A-scan) of the object **O** along the **z** direction. Particularly, according to the present invention, the processing unit **125** is configured tc receive from the optical detector **115** the sequence of digital samples of (the processed version of) the detected interference current signal and accordingly calculate for each predetermined depth among a set of predetermined depths along the **z** direction a corresponding reflectivity value.

According to an embodiment of the present invention, after the end of an A-scan, the optical scanner **120** is configured to be moved, *e.g.,* along the **x** direction, to a different target point in the **x-y** plane, so as to perform another A-scan.

According to an embodiment of the present invention, the processing unit **125** is configured to generate a cross-sectional image of the object **O** (referred to as B-scan) based on a set of A-scans associated with neighboring target points in the object **O**.

According to an embodiment of the present invention, the processing unit **125** is configured to generate a tridimensional (3D) image of the object **O** (referred to as C-scan) based on a set of B-scans through the **x-y** plane.

In the following, a single A-scan will be considered for ease of description.

The expression of the detected interference current signal is discussed here below, under the following hypotheses (which are satisfied in real SS-OCT systems):
- no relevant variation of the refraction index is present within the object **O**, so that no relevant curvature is present in the envelope of the Pointing vector and the light velocity can be assumed constant within the object **O**;
- the time needed to the light to traverse the object **O** is much smaller than any other considered time interval and, in particular, it is much smaller than the observation time;
- the complex wave vector of light (reflectivity and refraction index) does not depend on wavelength in the laser sweep bandwidth;
- the coherence length of the light source **105** is much longer than any length travelled by light within the object **O**;
- the linewidth of the light source **105** is negligible.

Moreover, in order to discuss the expression of the detected interference current, all the electric fields are assumed to be linearly polarized along the same polarization. As can be easily verified, this assumption provides a simplification that does not limit the validity of the discussion.

Assuming that the electric field emitted by the light source **105** is frequency modulated by a linear modulation in time t (sweep), the electric field frequency *f*(*t*)*,* the wave vector module *k*(*t*) in the object **O**, and the wave vector module *k*₀(*t*) in air can be written as: $f \left(t\right) = φ t + {f}_{0}$ (1) $k \left(t\right) = 2 π \frac{n f \left(t\right)}{c} = 2 π \left(\frac{n φ t}{c}+\frac{n {f}_{0}}{c}\right)$ (2) ${k}_{0} \left(t\right) = 2 π \frac{f \left(t\right)}{c} = 2 π \left(\frac{φ t}{c}+\frac{{f}_{0}}{c}\right)$ (3) where:
*φ* is the optical frequency sweep speed (generally measured in GHz/ns);
*φ t* is the optical frequency bandwidth;
*n* is the refraction index of the object **O**;
c the speed of light in vacuum, and
*f*₀ is the absolute optical frequency, that is the optical frequency at the beginning of the frequency sweep.

Assuming a perfectly balanced optical interferometer, the electric field ${{E}^{⇀}}_{R}$ received at the optical detector 115 from the reference arm 110(R) of the optical interferometer 110 can be written as: ${{E}^{⇀}}_{R} = A exp \left[-ι\left(2 π f\left(t\right)t-{k}_{0}\left(t\right)L\right)\right] \vec{p}$ (4) where 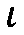 is the imaginary unit and *̅p̅*̅ is the polarization unit vector.

The electric field *E̅_{S}* received at the optical detector 115 from the operative arm 110(0) of the optical interferometer 110, and resulting from superposition of reflections from each layer of the object **O**, can be written as: ${\vec{E}}_{S} = A \vec{p} {\int }_{- {L}_{c} / 2}^{{L}_{c} / 2} \dot{r} \left(z\right) exp \left[-ι\left(2 π f\left(t\right)t-{k}_{0}\left(t\right)L-2 k\left(t\right)z\right)\right] dz$ (5) where *L_{c}* is the depth (along direction z) of the object **O**, and the average reflectivity ***r*** of the sample between spatial coordinates *z₁* and *z₂* along the z direction (with *z₂*>*z₁*) is given by: $\overline{r} = \frac{1}{{z}_{2} - {z}_{1}} {\int }_{z 1}^{z 2} \dot{r} \left(z\right) dz$ (6)

The detection currents generated by the photodiodes **115(I),115(Q)** (respectively denoted by *I_{I}*(*t*), *I_{Q}*(*t*)) take the following expressions: ${I}_{I} \left(t\right) = \frac{1}{2} μ \left[{\left|{{E}^{⇀}}_{R}\right|}^{2}+{\left|{{E}^{⇀}}_{s}\right|}^{2}+\left({{E}^{⇀}}_{R}{{E}^{⇀}}_{s}*-{{E}^{⇀}}_{R}*{{E}^{⇀}}_{s}\right)\right]$ (7a) ${I}_{Q} \left(t\right) = \frac{1}{2} μ \left[{\left|{{E}^{⇀}}_{R}\right|}^{2}+{\left|{{E}^{⇀}}_{s}\right|}^{2}-\left({{E}^{⇀}}_{R}{{E}^{⇀}}_{s}*-{{E}^{⇀}}_{R}*{{E}^{⇀}}_{s}\right)\right]$ (7b) where *µ* is the photodiode responsivity and * indicates the complex conjugate.

After subtraction between the detection currents *I_{I}*(*t*) and *I_{Q}*(*t*)*,* the only current term remaining is the beat between the electric field coming from both reference **110(R)** and operative **110(0)** arms of the optical interferometer **110,** i.e., the term containing the information about the A-scan and representing the detected interference current signal. Assuming ideal filtering and amplification, the detected interference current signal can be written as: $I \left(t\right) = {A}_{I} {\int }_{- {L}_{c} / 2}^{{L}_{c} / 2} \dot{r} \left(z\right) {rect}_{Θ} \left(t-\frac{Θ}{2}\right) cos \left[2 π φ \frac{2 n z}{c}t+ϕ\left(z\right)\right] dz$ (8) where:
*A_{I}* represents an amplitude of the detected interference current signal *I*(*t*)*,* and considers the overall analog power gain, the received amplitude, and photodiode efficiency;
*L_{c}* is the length of object **O** along direction **z;**
${rect}_{Θ} \left(t-\frac{Θ}{2}\right)$ is a function that considers the limitation of the detected interference current to a time interval [0, Θ];
*ϕ*(*z*) represents the phase component associated with each harmonic component of the detected interference current, and is given by: $ϕ \left(z\right) = 4 π \frac{n {f}_{0}}{c} z$ (9)

From the detected interference current signal *I*(*t*), the electrical bandwidth *Bₑ* and the Nyquist sampling rate *δt* can be derived as: ${B}_{e} = φ \frac{n {L}_{c}}{c}$ (10) $δt ≡ \frac{1}{2 {B}_{e}} ≡ \frac{c}{2 φ n {L}_{c}}$ (11)

Thus, A-scan reconstruction provides for determining the unknown function *ṙ*(*z*) from equation (8) where the detected interference current signal *I*(*t*) is known.

Equation (8) belongs to a class of integral equations called Fredholm equations of the first type (see, for example, Yuan, D., Zhang, X., "An overview of numerical reconstruction procedures for the first kind Fredholm integral equation", SN Appl. Sci. 1, 1178 (2019), https://doi.org/10.1007/s42452-019-1228-3).

Equation (8) in the standard form of the Fredholm equations may be written as: $I \left(t\right) = {A}_{I} {\int }_{- {L}_{c} / 2}^{{L}_{c} / 2} \dot{r} \left(z\right) H \left(t, z\right) dz$ (12) where: $H \left(z, t\right) = {A}_{I} {rect}_{Θ} \left(t-\frac{Θ}{2}\right) cos \left[2 π φ\frac{2 n z}{c}t+ϕ\left(z\right)\right]$ (13) represents the kernel, and is essentially a continuous set of sinusoidal functions depending on the parameter z observed for a limited time interval (i.e., the time interval [0, Θ]).

According to the present invention, the reconstruction procedure performed by the processing unit 125 is based on solving equation (8) in the time domain.

According to the invention, the procedure for solving equation (8) in the time domain provides for using a form of the so-called Singular Values Decomposition based on *"*Singular Values Decomposition" (Golub, Gene H., Van Loan, Charles F., (1996), "Matrix Computations" (3rd ed.), Johns Hopkins; Horn, Roger A., Johnson, Charles R., (1985), Section 7.3, "Matrix Analysis", Cambridge University Press. ISBN 978-0-521-38632-6).

According to an embodiment of the present invention, the procedure for solving equation (8) provides for a time discretization (which corresponds to a sampling of the detected interference current at a sampling rate *δt*, thereby obtaining a number q of time sampling points tₛ (s = 1, 2, ..., q)) and space discretization (i.e., a discretization of the spatial coordinate z into predetermined depths with a discretization step *δz*, thereby obtaining a number m of spatial sampling points zⱼ (j = 1, 2, ..., m)).

Indicating with bold capital letters a matrix and with underlined letters the algebraic vectors (to distinguish them from physical vectors, such as the polarization vector *̅p̅*̅ of equation (3)), then equation (12) may be written as: ${I}_{¯} = H {r}_{¯} dim \left({I}_{¯}\right) = q , dim \left({r}_{¯}\right) = m and dim \left(H\right) = q \times m$ (14) where:
q is the number of time sampling points tₛ (s = 1, 2, ..., q) in the time coordinate,
m is the number of spatial sampling points zⱼ (j = 1, 2, ..., m) in the space coordinate,
**H** is the equation Kernel calculated in the time and spatial samples, that is the kernel matrix,
l is a vector whose elements are current samples Iₛ (s = 1, 2, ..., q) of the detected interference current signal,
r is a vector whose elements are reflectivity samples rⱼ of (average) reflectivity (see equations (5) and (6)).

Assuming that the rank of the matrix **H** is maximum, due to the physical existence of a reflectivity distribution, the equation set (14), which is a system of linear equations, may allow determining the reflectivity at each sampling point.

The Singular Value Decomposition (SVD) of **H** provides for the following factorization: $H = U {H}_{D} V *$ (15) where U is a q x q complex unitary matrix, **H_{D}** is a q x m rectangular diagonal matrix with non-negative real numbers on the diagonal and **V** is a m x m complex unitary matrix. Such decomposition always exists for any complex matrix. Since in the considered case **H** is real, then **U** and **V** can be guaranteed to be real orthogonal matrixes.

The diagonal values σⱼ of the matrix **H_{D}** are the singular values of the matrix **H_{D}**, and the number of dᵢ that are different from zero is equal to the rank of **H_{D}**.

It is pointed out that the SVD is not unique, and it is always possible to choose the decomposition and the singular values σⱼ.

Solving the equation set (14) with the Singular Values Decomposition (SVD) method, the following expression can be obtained: ${r}_{¯} = V {H}_{D}^{- 1} {U}^{T} {I}_{¯}$ (16) where **V** and **U** are orthonormal matrixes implementing the linear transformation diagonalizing the Kernel H, the exponent -1 indicates the inverse conjugate, and ***H_{D}*** is the diagonal matrix, whose components are the singular values of the Kernel transformation.

Using the coordinate formalism, equation (16) can be written as: ${r}_{j} = {\sum }_{s = 1}^{q} {V}_{s , j} \frac{{I}_{s}}{{σ}_{s}} {U}_{j , s}^{T} j = 1 , … . m ; s = 1 , … , q$ (17) where σₛ are the singular values of the Kernel ***H*.** The singular values depend on the elements of the matrix, that are sinusoidal functions, as expressed by equation (13).

The phase component of these sinusoidal functions, which is given by equations (9) and (13), depends on the absolute optical frequency *f*₀ (which is much greater than the time-variant term carrying the useful information, *i.e.*, the term 2 *π φ* (2 n *z*)/*c*)). This implies a large number of periods of the sinusoidal function, thus any error (however small) in the phase component generates a large error in the evaluation of the sinusoidal function (this issue will be also referred to as phase instability).

Furthermore, because of unavoidable errors affecting the detected interference current signal (caused by noises and distortions as well as by numerical approximations), error terms εₛ are introduced in equation (16) affecting the current samples Iₛ: ${r}_{j} = {\sum }_{s = 1}^{q} {V}_{s , j} \frac{{I}_{s} - {ε}_{s}}{{σ}_{s}} {U}_{j , s}^{T} j = 1 , … . m ; s = 1 , … , q$ (18)

Since the solution for reflectivity must exist, we derive that, increasing the index s, the term *Iₛ* must decrease faster that the singular values, so that the ratio $\frac{{U}_{j , s}^{T} {I}_{s}}{{σ}_{s}}$ is definitely decreasing for each value of j (this means that a value of s exists so that for each s greater than that value this ratio decreases as a function of s). However, from equation (18) it can be inferred that the effect of error terms εₛ can be greatly amplified in case of small (close to zero) singular values σₛ, since the numerical error magnitude does not decrease with the index s and σₛ is at the denominator in equation (18), causing unpredictability in the solution (this issue will be also referred to as solution instability).

The phase and solution instabilities make the resolution of the Fredholm equation (12) an ill conditioned problem, where small variations in the initial data can lead to huge variations in the solution.

According to an embodiment of the present invention, the phase and/or error instabilities are faced as explained in the following.

The following two hypotheses are assumed:
- the solution of equation (8) is a continuous function with continuous derivatives at any order;
- the solution of equation (8) is not rapidly oscillating, i.e., a solution searched in a space discretization step *δz* has the solution spectrum with the highest spatial frequency that is smaller than 1/ *δz* (*e.g.,* six or seven times smaller.)

The first hypothesis is justified since the searched solution is a physical meaningful solution. The second hypothesis is justified by the fact that if said hypothesis was not true, the solution would be reconstructed with an insufficient spatial sampling.

According to an embodiment of the present invention, and under the two abovementioned hypotheses, Equation (8) is rewritten in discrete form to obtain the following system of equations: ${I}_{s} = {A}_{I} {\sum }_{j = 1}^{m} {r}_{j} cos \left[2 π φ\frac{2 n {z}_{j}}{c}{t}_{s}+ϕ\left({z}_{j}\right)\right]$ (19) where:
j = [1, 2, ..., m]
s = [1,2, ..., q]
zⱼ = z₁ + (j-1) *δz*
tₛ = t₁ + (s-1) *δt*
q ≥ m.

According to an embodiment of the present invention, the system of equations (19) is rewritten with the introduction of a new unknown variable x whose elements xᵥ depends on the phase term itself *ϕ*(*zⱼ*), *i.e.:* ${x}_{v} = \left\{\begin{matrix}{r}_{j} cos \left[ϕ\left({z}_{j}\right)\right] & v = 1 , … m \\ {r}_{j} sin \left[ϕ\left({z}_{j}\right)\right] & v = m + 1 , … 2 m\end{matrix}\right.$ (20)

By including the phase term *ϕ*(*zⱼ*) in the unknown variable of the system of equations, the issue of the phase instability can be advantageously solved.

According to an embodiment of the present invention, with the above definitions, and by accounting for the instability sources in the solution through a corresponding error vector ε, the system of equations (19) may be rewritten as: ${I}_{¯} = M {x}_{¯} + {ε}_{¯}$ (21) where the elements of the matrix **M** (i.e., the real coefficients of the system of equations) can be derived from the system of equations (18), and therefore depend on the refraction index n, the optical frequency sweep speed φ, the time sampling points tₛ and the spatial sampling points zⱼ.

According to the present invention, and assuming that matrix **M** is full rank, the system of equations (21) is solved by factorizing the matrix **M** through SVD, i.e., by calculating a matrix **D** that satisfies the following relationship: $M = U D {V}^{T}$ (22) where U and V are orthonormal matrixes since **M** is real-valued, and D is a diagonal matrix of singular values σ₁, σ₂, ..., σ₂ₘ.

Therefore, according to an embodiment of the present invention, the reflectivity values can be calculated as: ${r}_{¯} = V {D}^{- 1} {U}^{T} \left({I}_{¯}-{ε}_{¯}\right)$ (23)

Using the coordinate formalism, equation (22) can be written as: ${r}_{j} = {\sum }_{s = 1}^{q} {V}_{s , j} \frac{\left({I}_{s}-{ε}_{s}\right)}{{σ}_{s}} {U}_{j , s}^{T}$ (24)

As already mentioned above, the SVD is not unique, and it is always possible to choose the decomposition and the singular values dₛ. Particularly, according to an embodiment of the present invention, the singular values σₛ are ordered such that σₛ ≥ σₛ₊₁.

According to the present invention, in order to compensate the solution instability issue, amplificatior of the error terms εₛ is prevented by properly reducing the addends of equation (23) through the selection of a subset thereof. Particularly, according to the present invention, the summation in equation (23) is truncated ir order to consider only a subset of the received current samples *Iₛ*.

According to an embodiment of the present invention, the summation is truncated (performing a Truncated Singular Values Decomposition, TSVD) so as to keep only the addends corresponding to value of s lower than a corresponding truncation threshold W, *i.e.:* ${r}_{j} = {\sum }_{s = 1}^{W} {V}_{s , j} \frac{\left({I}_{s}-{ε}_{s}\right)}{{σ}_{s}} {U}_{j , s}^{T}$ (25) where the truncation threshold W is lower than q. In other words, according to an embodiment of the present invention, since the singular values σₛ are monotonically decreasing with respect to s so that that σₛ ≥ σₛ₊₁, by using equation (24) instead of equation (23), the reflectivity is calculated by considering only singular values σₛ that are higher than a singular value threshold SVTm = σ_{W} corresponding to the index s equal to the truncation threshold. According to an embodiment of the present invention, the singular value threshold SVTm is equal to a fraction of the highest singular value σₛ₌₁ among the singular values σₛ obtainable by the factorization of the matrix **M.**

In this way, by discarding the addends that correspond to small singular values σₛ, amplification of the error terms εₛ by too small singular values σₛ at the denominator is advantageously prevented, or at least reduced.

According to an embodiment of the present invention, the truncation threshold Wis set to a value sufficiently low so as to discard the singular values σₛ sufficiently close to zero and at the same time to a value sufficiently large to obtain an acceptable approximation of the reflectivity.

It can be demonstrated that a value of the truncation threshold *W* which satisfies these two contrasting requirements exists (and it is not unique) if the so-called discrete Picard condition (Per Christian Hansen, "Truncated Singular Value Decomposition Solutions to Discrete III-Posed Problems with III-Defermined Numerical Rank", SIAM J. Scl. Stat. Comput., Vol 11, No.3, pages 503-518, May 1990) is satisfied, i.e., if $\frac{{U}_{j , s}^{T} {I}_{s} - {U}_{j , s + 1}^{T} {I}_{s + 1}}{{σ}_{s} - {σ}_{s + 1}} \frac{{σ}_{s}}{{U}_{j , s}^{T} {I}_{s}} > 1 ∨ s > {s}_{0} W ≫ {s}_{0}$ (26)

The meaning of the Picard condition is that the term ${U}_{j , s}^{T}$ *Iₛ* decreases more rapidly than the singular values *σₛ* as a function of s and it is satisfied in all the interesting cases for SS-OCT due to the fact that the reflectivity function physically exists.

According to an embodiment of the present invention, the truncation threshold W has to be evaluated case-by-case depending on the matrix **M,** and therefore based on the refraction index n, the range of frequency sweep, the optical frequency sweep speed φ, the time sampling points tₛ and the spatial sampling points zⱼ. Applicant has also verified that the truncation threshold W does not depend on the details of r, so that it can be determined a-priori once the matrix **M** is known if the qualitative characteristics of the expected solutions are known.

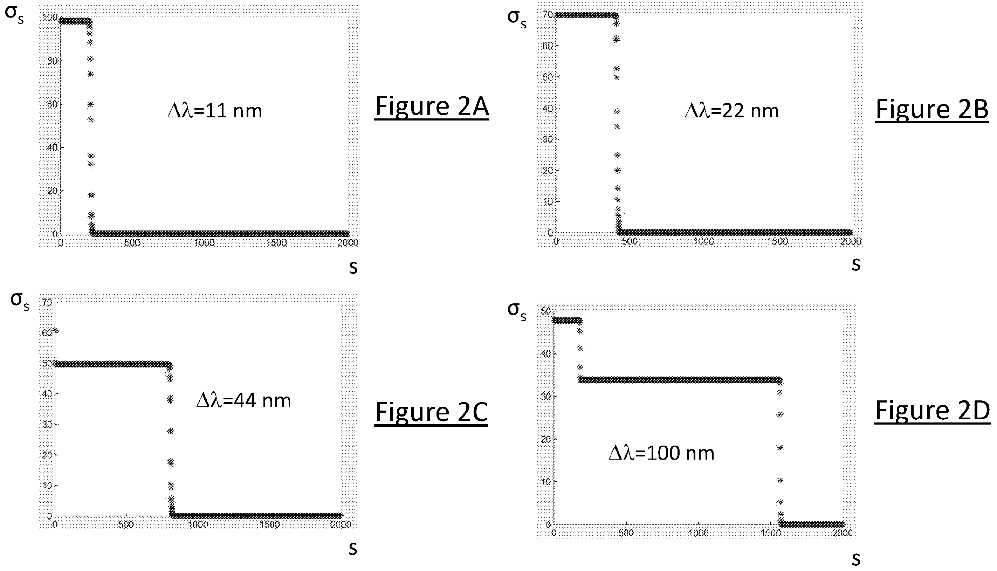

**Figures 2A-2D** are diagrams showing four examples of sets of singular values σₛ as a function of s obtained by factorizing a matrix **M** for SS-OCT applications through SVD corresponding to four different frequency sweeps of the light source **105.** **Figure 2A** discloses a set of singular values σₛ as a function of s obtained by factorizing a matrix **M** for SS-OCT applications through SVD when the wavelength of the coherent light signal generated by the light source **105** is swept across a wavelength sweep interval *Δλ* = 11 nm. In this case, the singular values σₛ become very close to zero starting from about s = 220. According to an embodiment of the present invention, a candidate for being selected as truncation threshold W is around the value s = 220, so as to have a corresponding singular value threshold SVTm lower than 90, such as for example 40.

**Figure 2B** discloses a set of singular values σₛ as a function of s obtained by factorizing a matrix **M** for SS-OCT applications through SVD when the wavelength of the coherent light signal generated by the light source **105** is swept across a wavelength sweep interval *Δλ* = 22 nm. In this case, the singular values σₛ become very close to zero starting from about s = 480. According to an embodiment of the present invention, a candidate for being selected as truncation threshold W is around the value s = 480, so as to have a corresponding singular value threshold SVTm lower than 65, such as for example 20.

**Figure 2C** discloses a set of singular values σₛ as a function of s obtained by factorizing a matrix M for SS-OCT applications through SVD when the wavelength of the coherent light signal generated by the light source **105** is swept across a wavelength sweep interval *Δλ* = 44 nm. In this case, the singular values σₛ become very close to zero starting from about s = 800. According to an embodiment of the present invention, a candidate for being selected as truncation threshold W is around the value s = 800, so as to have a corresponding singular value threshold SVTm lower than 45, such as for example 5.

**Figure 2D** discloses a set of singular values σₛ as a function of s obtained by factorizing a matrix **M** for SS-OCT applications through SVD when the wavelength of the coherent light signal generated by the light source **105** is swept across a wavelength sweep interval *Δλ* = 100 nm. In this case, the singular values σₛ become very close to zero starting from about s = 1600. According to an embodiment of the present invention, a candidate for being selected as truncation threshold W is around the value s = 1600, so as to have a corresponding singular value threshold SVTm lower than 35, such as for example 31.

Applicant has observed that the singular values σₛ become very close to zero starting from a value of s that depends on the wavelength sweep interval *Δλ*. The larger the wavelength sweep interval *Δλ*, the larger the value of s from which the singular values σₛ become very close to zero. Moreover, Applicant has observed that the value of the first singular value σₛ of the set (*i.e.*, the one corresponding to s = 1) decreases as the wavelength sweep interval *Δλ* increases.

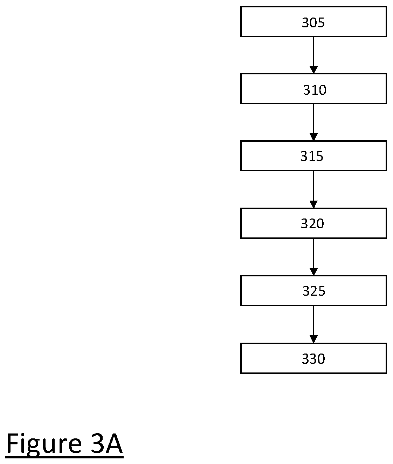

**Figure 3A** is a flowchart illustrating the operations performed by the processing unit **125** for the generation of an A-scan of the object **O** (see **Figure 1****)** along the **z** direction according to an embodiment of the present invention.

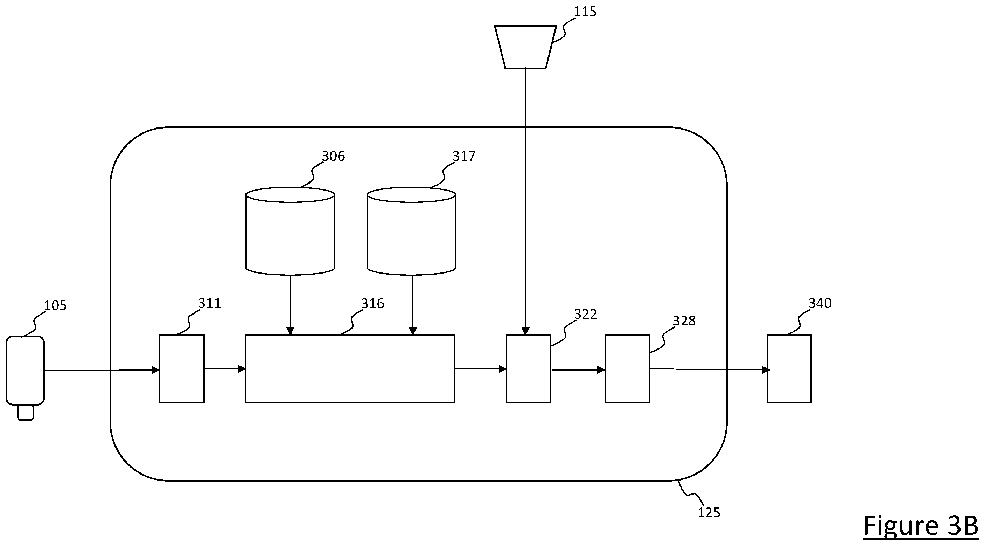

**Figure 3B** illustrates in terms of functional blocks modules of the processing units **125** involved in operations performed by the processing unit **125** for the generation of an A-scan of the object **O** according to an embodiment of the present invention.

With joint reference to **Figures 3A-3B****,** the first operation (flow chart block **305)** according to an embodiment of the present invention provides for having the processing unit **125** retrieve the matrix **M** corresponding to the particular kind of object **O** to be scanned (*e.g.*, the retinal zone of an eye).

For this purpose, according to an embodiment of the present invention, the processing unit **125** accesses a memory unit module **306** of the processing unit **125** storing data representing the singular values σ₁, σ₂, ..., σ₂ₘ of the diagonal matrix **D** corresponding to matrix **M** as well as the elements of the corresponding orthonormal matrixes **U** and **V** (see equation (22)). According to an embodiment of the present invention, the memory unit module **306** of the the processing unit **125** stores data representing the singular values σ₁, σ₂, ..., σ₂ₘ of the diagonal matrix **D** (as well as the elements of the corresponding orthonormal matrixes **U** and **V)** of a plurality of different matrixes **M,** each one corresponding to a different kind of object **O**.

Alternative solutions are also contemplated, in which the data representing the singular values σ₁, σ₂, ..., σ₂ₘ of the diagonal matrix **D** corresponding to matrix **M** and the elements of the corresponding orthonormal matrixes **U** and **V** is remotely sent to the the processing unit **125** and/or submitted by a user of the system **100.**

According to an embodiment of the present invention, the processing unit **125** retrieves information about the wavelength sweep interval *Δλ* used by the light source **105** for the frequency sweep of the coherent light signal (flow chart block **310).** For this purpose, according to an embodiment of the present invention, the processing unit **125** comprises a sweep identifier module **311** configured to receive data representing the wavelength sweep interval *Δλ* employed by the light source **105** from the light source **105** itself or from a user of the system **100.**

Then, according to an embodiment of the present invention, the processing unit **125** selects (flow chart block **315)** a truncation threshold W based on the (retrieved data corresponding to the) matrix **M** and/or on the (retrieved data corresponding to the) wavelength sweep interval *Δλ* used by the light source **105.**

For this purpose, according to an embodiment of the present invention, the processing unit **125** comprises a threshold selector module **316** configured to select the truncation threshold W exploiting predefined look-up tables and/or mathematical/logical/empirical formulas depicting relationships between values of the truncation threshold W and values of the matrix **M** (particularly, of singular values σₛ of the matrix **M)** and/or of the wavelength sweep interval *Δλ*. According to an embodiment of the present invention, data representing said predefined look-up tables and/or mathematical/logical/empirical formulas is stored in a corresponding relationship storage module **317** of the processing unit **125.** Alternative solutions are also contemplated, in which said data is remotely sent to the the processing unit **125** and/or submitted by a user of the system **100.**

Then, according to an embodiment of the present invention, the processing unit **125** collects (flow chart block **320)** the sequence of current samples Iₛ (s = 1, 2, ..., q) of the detected interference current signal generated by the optical detector **115** in response to the sweep carried out by the light source **105.**

At this point, according to the present invention, a reflectivity vector generator module **322** of the processing unit **125** generates (flow chart block **325)** a reflectivity vector r = [r₁, r₂, ..., rⱼ, ..., rₘ], in which the generic element rⱼ of the reflectivity vector *r* is a reflectivity value indicative of the reflectivity of the object **O** at the predetermined depth zⱼ and is calculated by the processing unit **125** using equation (25), *i.e.:* ${r}_{j} = {Σ}^{W} {V}_{s , j} \frac{\left({I}_{s}\right)}{{σ}_{s}} {U}_{j , s}^{T}$

Then, according to the present invention, a A-scanner module **328** of the processing unit **125** generates (flow chart block **330)** an A-scan of the object **O** along the **z** direction based on the elements rⱼ of the reflectivity vector *r*. According to an embodiment of the present invention, a depiction of the A-scan outputted by the processing unit **125** may be provided by a peripheral module **340** coupled to the processing unit **125,** such as a display device or a printer device. In addition or in alternative, data representing the A-scan outputted by the processing unit **125** may be stored in a storage unit.

According to an embodiment, one or more additional techniques, may complement the procedure performed by the processing unit **125** to improve performance.

According to an embodiment, one or more post-processing techniques may complement the procedure performed by the processing unit **125** to improve performance.

Just as an example, a post-processing technique based on reflectivity value linear transformation or based on reflectivity value power transformation may be used. These are examples of contrast enhancement algorithms that can be used in image processing systems.

In case of linear transformation, new reflectivity values *r̅ⱼ* are evaluated through a linear transformation starting from the reflectivity values *rⱼ*, *i.e.:* ${\overline{r}}_{j} = {C}_{1} {r}_{j} + {C}_{0}$

In case of power transformation, new reflectivity values *r̅ⱼ* are evaluated through a power transformation starting from the reflectivity values *rⱼ*, *i.e.:* ${\overline{r}}_{j} = {C}_{1} {{r}_{j}}^{{C}_{0}}$

In both cases *C*₁ and *C*₀ are real constants, to be determined a priori on the ground of the expected type of the detected interference signal.

In order to test the performances of the procedures for the calculation of the reflectivity according to the embodiments of the present invention, the image resolution obtainable using the calculated reflectivity is determined using a couple of space pulses at a given distance one from the other. By image resolution is herein intended the minimum distance between the nearest space pulses that can be distinguished in the image.

The following parameters have been used to determine the image resolution.

| Image Parameters | | |
|---|---|---|
| Image depth | 2 | mm |
| Pulses width | 5 | 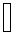 m |
| Refraction index | 1.35 | ---- |
| Pulses shape | Gaussian | ---- |

| SS-OCT Parameters | | |
|---|---|---|
| A-Scan duration | 1 | 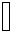 s |
| Sweep shape | Linear | ---- |
| Mid wavelength | 1064 | nm |
| Sampling rate | 0.5 | ns |

| Algorithm Parameters | | |
|---|---|---|
| Depth step | 1 | 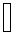 m |
| Threshold SV | 40 | ---- |
| Postprocessing | NO | ---- |

Two parameters are defined: a minimum distance d_{M} at which the signal reaches one quarter of the pulses amplitude in between the adjacent pulses and the minimum distance dₛ at which the pulses can be easily distinguished, that is the signal in between the pulses is half the pulse amplitude.

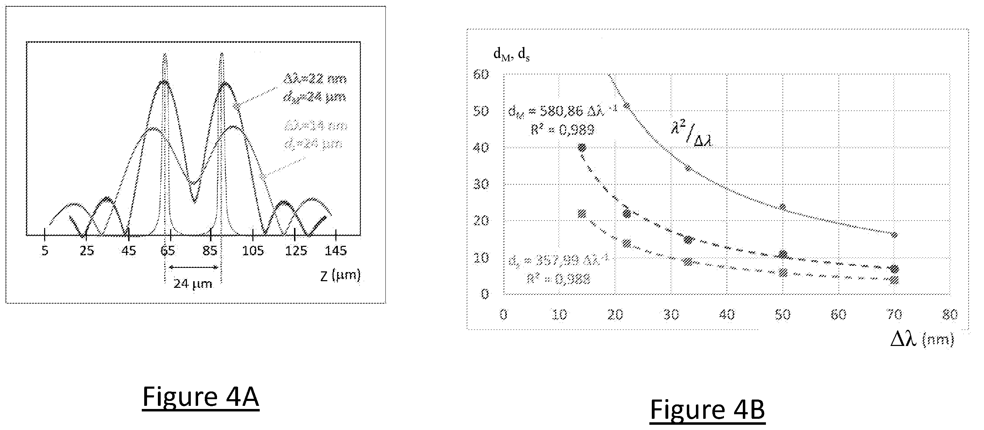

An example showing the meaning of these definitions is provided in **Figure 4A****.** In the case illustrated in **Figure 4A****,** the pulse distance is 22 µm and the response of the procedure according to the embodiments of the invention is shown in the case of two different values of Δλ. For Δλ = 22 nm, d_{M} = 24 µm, and for Δλ 14 nm, dₛ = 24 µm.

A general idea of the ability of using TSVD in the procedure according to the embodiments of the present invention for distinguishing nearby pulses can be obtained by looking at **Figure 4B** where a couple of Gaussian pulses are considered. **Figure 4B** shows d_{M} and dₛ versus Δλ using the parameters of the previous table. As a reference also the curve ${λ}_{0}^{2} / Δλ$ is reported.

Naturally, in order to satisfy local and specific requirements, a person skilled in the art may apply to the disclosure described above many logical and/or physical modifications and alterations. More specifically, although the present disclosure has been described with a certain degree of particularity with reference to preferred embodiments thereof, it should be understood that various omissions, substitutions and changes in the form and details as well as other embodiments are possible, as long as the modified embodiments stay within the scope of the invention as defined in the appended claims.

For example, although in the present description the reference has made to the reconstruction of an A-scan starting from a detected interference current signal, the concepts of the present invention can be directly applied to other detected interference electrical signals, such as a detected interference voltage signal.

## Claims

1. An Optical Coherence Tomography (OCT) system **(100)** comprising:
a light source **(105)** configured to generate a coherent light signal having a wavelength that varies over time across a time interval;
an optical interferometer **(110)** configured to divide the coherent light signal into a reference forward signal directed to a reference arm of the optical interferometer leading to a reference reflector **(R),** and into an operative forward signal directed to an operative arm of the optical interferometer leading to a target object (**O**);
an optical detector **(115)** configured to detect an interference light signal generated by a combination of a reference return signal returning from the reference arm after reflection by the reference reflector **(R)** and an operative return signal returning from the operative arm after reflection by the object (**O**), and configured to generate a corresponding detected interference electrical signal indicative of the interference light signal;
a processing unit **(125)** configured to:
- receive from the optical detector **(115)** a sequence of electrical signal samples of the detected interference electrical signal collected in corresponding sample times within the time interval;
- process the received electrical signal samples to calculate for each predetermined depth among a set of predetermined depths inside the target object a corresponding reflectivity value indicative of the reflectivity of the target object at said predetermined depth, so as to obtain a corresponding set of reflectivity values;
wherein:
the processing unit **(125)** is configured to calculate the reflectivity values of the set by calculating each reflectivity value as a corresponding summation of electrical signal samples each one weighted by a respective weight, wherein said weights corresponds to singular values obtainable by the decomposition of a coefficient matrix of a system of equations, each equation of the system expressing a relationship between one of the received electrical signal samples and at least one of the reflectivity values of the set, wherein said summation involves a subset of the received electrical signal samples,
wherein said reflectivity values are calculated as a reflectivity vector in which each element of the reflectivity vector is a corresponding one of said reflectivity values,
and wherein the processing unit is configured to generate an A-scan of the target object based on the elements of said reflectivity vector.

2. The OCT system **(100)** of claim 1, wherein each weight corresponds to the inverse of a respective one of said singular values.

3. The OCT system **(100)** of any of the preceding claims, wherein said coefficient matrix depends on at least one among:
- refraction index of the target object (**O**);
- the sampling times;
- the predetermined depths;
- a rate of variation over time of the wavelength of coherent light signal.

4. The OCT system **(100)** of any of the preceding claims, wherein:
- each singular value corresponding to a respective electrical signal sample, apart from the singular value corresponding to the first electrical signal sample of the sequence, is not higher than the singular value corresponding to the previous electrical signal sample in the sequence,
- the processing unit **(125)** is configured to select said subset of the received electrical signal samples by selecting electrical signal samples corresponding to singular values higher than a threshold.

5. The OCT system **(100)** of claim 4, wherein said threshold is equal to a fraction of the highest singular value among the singular values obtainable by the decomposition of said coefficient matrix.

6. The OCT system **(100)** of any of the preceding claims, wherein the processing unit **(125)** is configured to select said subset of the received electrical signal samples by selecting, among all the electrical signal samples of the sequence, a subsequence comprising initial electrical signal samples in the sequence and by discarding subsequent electrical signal samples in the sequence.

7. The OCT system **(100)** of any of the preceding claims, wherein said subset of the received electrical signal samples comprises a number of the received electrical signal samples, said number depending on the difference between the highest and lower wavelength of the coherent light signal during said time interval.

8. The OCT system **(100)** of any of the preceding claims, wherein the unknown variables of said system of linear equations comprise the reflectivity values of the set multiplied by a term depending on phase values of said electrical signal samples, said phase values being functions of said predetermined depths.

9. The OCT system **(100)** of any of the preceding claims, wherein the processing unit is configured to generate an A-scan of the target object (**O**) based on the set of the reflectivity values.

10. The OCT system **(100)** of any of the preceding claims, wherein:
- the target object (**O**) is a retinal zone of an eye, and
- the wavelength of the coherent light spans, during said time interval, a wavelength sweep interval comprising a wavelength corresponding to 1064 nm.

11. The OCT system **(100)** of claim 10, wherein:
- the difference between the highest and lower wavelength of the coherent light in said wavelength sweep interval corresponds to 11 nm, and the processing unit **(125)** is configured to select said subset of the received electrical signal samples by selecting current samples corresponding to singular values higher than a threshold lower than 90, preferably corresponding to 40, or
- the difference between the highest and lower wavelength of the coherent light in said wavelength sweep interval corresponds to 22 nm, and the processing unit **(125)** is configured to select said subset of the received electrical signal samples by selecting current samples corresponding to singular values higher than a threshold lower than 65, preferably corresponding to 20, or
- the difference between the highest and lower wavelength of the coherent light in said wavelength sweep interval corresponds to 44 nm, and the processing unit **(125)** is configured to select said subset of the received electrical signal samples by selecting current samples corresponding to singular values higher than a threshold lower than 45, preferably corresponding to 5, or
- the difference between the highest and lower wavelength of the coherent light in said wavelength sweep interval corresponds to 110 nm, and the processing unit **(125)** is configured to select said subset of the received electrical signal samples by selecting current samples corresponding to singular values higher than a threshold lower than 40, preferably corresponding to 31.

12. A method for carrying out an Optical Coherence Tomography (OCT) on a target object (**O**), the method comprising:
- generating a coherent light signal having a wavelength that varies over time across a time interval;
- dividing the coherent light signal into a reference forward signal directed to a reference reflector **(R),** and into an operative forward signal directed to the target object (**O**);
- detecting an interference light signal generated by a combination of a reference return signal returning from the reference reflector **(R)** and an operative return signal returning from the object (**O**);
- generating a detected interference electrical signal indicative of the interference light signal, wherein said generating a detected interference electrical signal comprises:
- receiving a sequence of electrical signal samples of the detected interference electrical signal collected in corresponding sample times within the time interval;
- processing the received electrical signal samples to calculate for each predetermined depth among a set of predetermined depths inside the target object a corresponding reflectivity value indicative of the reflectivity of the target object at said predetermined depth, so as to obtain a corresponding set of reflectivity values, wherein said processing the received electrical signal samples comprises:
- calculating each reflectivity value as a corresponding summation of electrical signal samples each one weighted by a respective weight, wherein said weights corresponds to singular values obtainable by the decomposition of a coefficient matrix of a system of equations, each equation of the system expressing a relationship between one of the received electrical signal samples and at least one of the reflectivity values of the set, and wherein said summation involves a subset of the received electrical signal samples, the method further comprising:
- generating a reflectivity vector in which each element of the reflectivity vector is a corresponding one of said reflectivity values of the set of reflectivity values; and
- generating an A-scan of the target object based on the elements of said reflectivity vector.

## Patentansprüche

1. System (**100**) zur optischen Kohärenztomographie (OCT), umfassend:
eine Lichtquelle (**105**), die dazu ausgebildet ist, ein kohärentes Lichtsignal zu erzeugen, das eine Wellenlänge aufweist, die sich über ein Zeitintervall hinweg mit der Zeit ändert;
ein optisches Interferometer (**110**), das dazu ausgebildet ist, das kohärente Lichtsignal in ein Referenz-Hinlaufsignal, das zu einem Referenzarm des optischen Interferometers gerichtet ist, der zu einem Referenzreflektor (**R**) führt, und in ein operatives Hinlaufsignal zu teilen, das zu einem operativen Arm des optischen Interferometers gerichtet ist, der zu einem Zielobjekt (**O**) führt;
einen optischen Detektor (**115**), der dazu ausgebildet ist, ein Interferenzlichtsignal zu detektieren, das durch eine Kombination eines Referenz-Rücklaufsignals, das nach Reflexion durch den Referenzreflektor (**R**) von dem Referenzarm zurückkehrt, und eines operativen Rücklaufsignals erzeugt wird, das nach Reflexion durch das Objekt (**O**) von dem operativen Arm zurückkehrt, und der dazu ausgebildet ist, ein entsprechendes detektiertes elektrisches Interferenzsignal zu erzeugen, das für das Interferenzlichtsignal kennzeichnend ist;
eine Verarbeitungseinheit (**125**), die dazu ausgebildet ist:
- von dem optischen Detektor (**115**) eine Folge von elektrischen Signalabtastwerten des detektierten elektrischen Interferenzsignals zu empfangen, die zu entsprechenden Abtastzeitpunkten innerhalb des Zeitintervalls erfasst wurden;
- die empfangenen elektrischen Signalabtastwerte zu verarbeiten, um für jede vorbestimmte Tiefe aus einem Satz von vorbestimmten Tiefen innerhalb des Zielobjekts einen entsprechenden Reflexivitätswert zu berechnen, der für die Reflexivität des Zielobjekts bei der vorbestimmten Tiefe kennzeichnend ist, um einen entsprechenden Satz von Reflexivitätswerten zu erhalten;
wobei:
die Verarbeitungseinheit (**125**) dazu ausgebildet ist, die Reflexivitätswerte des Satzes zu berechnen, indem jeder Reflexivitätswert als eine entsprechende Summe von elektrischen Signalabtastwerten berechnet wird, von denen jeder mit einem jeweiligen Gewicht gewichtet ist, wobei die Gewichte Singularwerten entsprechen, die durch die Zerlegung einer Koeffizientenmatrix eines Gleichungssystems erhalten werden können, wobei jede Gleichung des Systems eine Beziehung zwischen einem der empfangenen elektrischen Signalabtastwerte und mindestens einem der Reflexivitätswerte des Satzes ausdrückt, wobei die Summe eine Teilmenge der empfangenen elektrischen Signalabtastwerte umfasst,
wobei die Reflexivitätswerte als ein Reflexivitätsvektor berechnet werden, in dem jedes Element des Reflexivitätsvektors einem entsprechenden der Reflexivitätswerte entspricht,
und wobei die Verarbeitungseinheit dazu ausgebildet ist, einen A-Scan des Zielobjekts auf Grundlage der Elemente des Reflexivitätsvektors zu erzeugen.

2. OCT-System (**100**) nach Anspruch 1, wobei jedes Gewicht dem Kehrwert eines jeweiligen der Singularwerte entspricht.

3. OCT-System (**100**) nach einem der vorhergehenden Ansprüche, wobei die Koeffizientenmatrix von mindestens einem der folgenden Parameter abhängt:
- Brechungsindex des Zielobjekts (**O**);
- den Abtastzeitpunkten;
- den vorbestimmten Tiefen;
- einer zeitlichen Änderungsrate der Wellenlänge des kohärenten Lichtsignals.

4. OCT-System (**100**) nach einem der vorhergehenden Ansprüche, wobei:
- jeder Singularwert, der einem jeweiligen elektrischen Signalabtastwert entspricht, mit Ausnahme des Singularwerts, der dem ersten elektrischen Signalabtastwert der Folge entspricht, nicht höher als der Singularwert ist, der dem vorhergehenden elektrischen Signalabtastwert in der Folge entspricht,
- die Verarbeitungseinheit (**125**) dazu ausgebildet ist, die Teilmenge der empfangenen elektrischen Signalabtastwerte auszuwählen, indem elektrische Signalabtastwerte ausgewählt werden, die Singularwerten entsprechen, die höher als ein Schwellenwert sind.

5. OCT-System (**100**) nach Anspruch 4, wobei der Schwellenwert gleich einem Bruchteil des höchsten Singularwerts unter den Singularwerten ist, die durch die Zerlegung der Koeffizientenmatrix erhalten werden können.

6. OCT-System (**100**) nach einem der vorhergehenden Ansprüche, wobei die Verarbeitungseinheit (**125**) dazu ausgebildet ist, die Teilmenge der empfangenen elektrischen Signalabtastwerte auszuwählen, indem aus allen elektrischen Signalabtastwerten der Folge eine Teilfolge ausgewählt wird, die anfängliche elektrische Signalabtastwerte in der Folge umfasst, und indem nachfolgende elektrische Signalabtastwerte in der Folge verworfen werden.

7. OCT-System (**100**) nach einem der vorhergehenden Ansprüche, wobei die Teilmenge der empfangenen elektrischen Signalabtastwerte eine Anzahl der empfangenen elektrischen Signalabtastwerte umfasst, wobei die Anzahl von der Differenz zwischen der höchsten und der niedrigeren Wellenlänge des kohärenten Lichtsignals während des Zeitintervalls abhängt.

8. OCT-System (**100**) nach einem der vorhergehenden Ansprüche, wobei die unbekannten Variablen des Systems linearer Gleichungen die Reflexivitätswerte des Satzes, multipliziert mit einem Term, der von Phasenwerten der elektrischen Signalabtastwerte abhängt, umfassen, wobei die Phasenwerte Funktionen der vorbestimmten Tiefen sind.

9. OCT-System (**100**) nach einem der vorhergehenden Ansprüche, wobei die Verarbeitungseinheit dazu ausgebildet ist, einen A-Scan des Zielobjekts (**O**) auf Grundlage des Satzes der Reflexivitätswerte zu erzeugen.

10. OCT-System (**100**) nach einem der vorhergehenden Ansprüche, wobei:
- das Zielobjekt (**O**) eine Netzhautzone eines Auges ist, und
- die Wellenlänge des kohärenten Lichts während des Zeitintervalls ein Wellenlängen-Sweep-Intervall durchläuft, das eine Wellenlänge entsprechend 1064 nm umfasst.

11. OCT-System (**100**) nach Anspruch 10, wobei:
- die Differenz zwischen der höchsten und der niedrigeren Wellenlänge des kohärenten Lichts in dem Wellenlängen-Sweep-Intervall 11 nm entspricht, und die Verarbeitungseinheit (**125**) dazu ausgebildet ist, die Teilmenge der empfangenen elektrischen Signalabtastwerte auszuwählen, indem aktuelle Abtastwerte ausgewählt werden, die Singularwerten entsprechen, die höher als ein Schwellenwert von weniger als 90, vorzugsweise entsprechend 40, sind, oder
- die Differenz zwischen der höchsten und der niedrigeren Wellenlänge des kohärenten Lichts in dem Wellenlängen-Sweep-Intervall 22 nm entspricht, und die Verarbeitungseinheit (**125**) dazu ausgebildet ist, die Teilmenge der empfangenen elektrischen Signalabtastwerte auszuwählen, indem aktuelle Abtastwerte ausgewählt werden, die Singularwerten entsprechen, die höher als ein Schwellenwert von weniger als 65, vorzugsweise entsprechend 20, sind, oder
- die Differenz zwischen der höchsten und der niedrigeren Wellenlänge des kohärenten Lichts in dem Wellenlängen-Sweep-Intervall 44 nm entspricht, und die Verarbeitungseinheit (**125**) dazu ausgebildet ist, die Teilmenge der empfangenen elektrischen Signalabtastwerte auszuwählen, indem aktuelle Abtastwerte ausgewählt werden, die Singularwerten entsprechen, die höher als ein Schwellenwert von weniger als 45, vorzugsweise entsprechend 5, sind, oder
- die Differenz zwischen der höchsten und der niedrigeren Wellenlänge des kohärenten Lichts in dem Wellenlängen-Sweep-Intervall 110 nm entspricht, und die Verarbeitungseinheit (**125**) dazu ausgebildet ist, die Teilmenge der empfangenen elektrischen Signalabtastwerte auszuwählen, indem aktuelle Abtastwerte ausgewählt werden, die Singularwerten entsprechen, die höher als ein Schwellenwert von weniger als 40, vorzugsweise entsprechend 31, sind.

12. Verfahren zum Durchführen einer optischen Kohärenztomographie (OCT) an einem Zielobjekt (**O**), wobei das Verfahren umfasst:
- Erzeugen eines kohärenten Lichtsignals, das eine Wellenlänge aufweist, die sich über ein Zeitintervall hinweg mit der Zeit ändert;
- Teilen des kohärenten Lichtsignals in ein Referenz-Hinlaufsignal, das zu einem Referenzreflektor (**R**) gerichtet ist, und in ein operatives Hinlaufsignal, das zu dem Zielobjekt (**O**) gerichtet ist;
- Detektieren eines Interferenzlichtsignals, das durch eine Kombination eines Referenz-Rücklaufsignals, das von dem Referenzreflektor (**R**) zurückkehrt, und eines operativen Rücklaufsignals erzeugt wird, das von dem Objekt (**O**) zurückkehrt;
- Erzeugen eines detektierten elektrischen Interferenzsignals, das für das Interferenzlichtsignal kennzeichnend ist, wobei das Erzeugen eines detektierten elektrischen Interferenzsignals umfasst:
- Empfangen einer Folge von elektrischen Signalabtastwerten des detektierten elektrischen Interferenzsignals, die zu entsprechenden Abtastzeitpunkten innerhalb des Zeitintervalls erfasst wurden;
- Verarbeiten der empfangenen elektrischen Signalabtastwerte, um für jede vorbestimmte Tiefe aus einem Satz von vorbestimmten Tiefen innerhalb des Zielobjekts einen entsprechenden Reflexivitätswert zu berechnen, der für die Reflexivität des Zielobjekts bei der vorbestimmten Tiefe kennzeichnend ist, um einen entsprechenden Satz von Reflexivitätswerten zu erhalten, wobei das Verarbeiten der empfangenen elektrischen Signalabtastwerte umfasst:
- Berechnen jedes Reflexivitätswerts als eine entsprechende Summe von elektrischen Signalabtastwerten, von denen jeder mit einem jeweiligen Gewicht gewichtet ist, wobei die Gewichte Singularwerten entsprechen, die durch die Zerlegung einer Koeffizientenmatrix eines Gleichungssystems erhalten werden können, wobei jede Gleichung des Systems eine Beziehung zwischen einem der empfangenen elektrischen Signalabtastwerte und mindestens einem der Reflexivitätswerte des Satzes ausdrückt, und wobei die Summe eine Teilmenge der empfangenen elektrischen Signalabtastwerte umfasst, wobei das Verfahren ferner umfasst:
- Erzeugen eines Reflexivitätsvektors, in dem jedes Element des Reflexivitätsvektors einem entsprechenden der Reflexivitätswerte des Satzes von Reflexivitätswerten entspricht; und
- Erzeugen eines A-Scans des Zielobjekts auf Grundlage der Elemente des Reflexivitätsvektors.

## Revendications

1. Système (**100**) de tomographie par cohérence optique (OCT) comprenant :
une source de lumière (**105**) configurée pour générer un signal lumineux cohérent ayant une longueur d'onde qui varie dans le temps sur un intervalle de temps ;
un interféromètre optique (**110**) configuré pour diviser le signal lumineux cohérent en un signal aller de référence dirigé vers un bras de référence de l'interféromètre optique conduisant à un réflecteur de référence (**R**), et en un signal aller opératif dirigé vers un bras opératif de l'interféromètre optique conduisant à un objet cible (**O**) ;
un détecteur optique (**115**) configuré pour détecter un signal lumineux d'interférence généré par une combinaison d'un signal retour de référence revenant du bras de référence après réflexion par le réflecteur de référence (**R**) et d'un signal retour opératif revenant du bras opératif après réflexion par l'objet (**O**), et configuré pour générer un signal électrique d'interférence détecté correspondant, indicatif du signal lumineux d'interférence ;
une unité de traitement (**125**) configurée pour :
- recevoir du détecteur optique (**115**) une séquence d'échantillons de signal électrique du signal électrique d'interférence détecté collectés à des instants d'échantillonnage correspondants à l'intérieur de l'intervalle de temps ;
- traiter les échantillons de signal électrique reçus pour calculer, pour chaque profondeur prédéterminée parmi un ensemble de profondeurs prédéterminées à l'intérieur de l'objet cible, une valeur de réflectivité correspondante indicative de la réflectivité de l'objet cible à ladite profondeur prédéterminée, de manière à obtenir un ensemble correspondant de valeurs de réflectivité ;
dans lequel :
l'unité de traitement (**125**) est configurée pour calculer les valeurs de réflectivité de l'ensemble en calculant chaque valeur de réflectivité sous la forme d'une sommation correspondante d'échantillons de signal électrique, chacun étant pondéré par un poids respectif, dans lequel lesdits poids correspondent à des valeurs singulières pouvant être obtenues par la décomposition d'une matrice de coefficients d'un système d'équations, chaque équation du système exprimant une relation entre l'un des échantillons de signal électrique reçus et au moins l'une des valeurs de réflectivité de l'ensemble, dans lequel ladite sommation implique un sous-ensemble des échantillons de signal électrique reçus,
dans lequel lesdites valeurs de réflectivité sont calculées sous la forme d'un vecteur de réflectivité dans lequel chaque élément du vecteur de réflectivité est une valeur correspondante parmi lesdites valeurs de réflectivité,
et dans lequel l'unité de traitement est configurée pour générer un A-scan de l'objet cible sur la base des éléments dudit vecteur de réflectivité.

2. Système OCT (**100**) selon la revendication 1, dans lequel chaque poids correspond à l'inverse d'une valeur respective parmi lesdites valeurs singulières.

3. Système OCT (**100**) selon l'une quelconque des revendications précédentes, dans lequel ladite matrice de coefficients dépend d'au moins un parmi :
- l'indice de réfraction de l'objet cible (**O**) ;
- les instants d'échantillonnage ;
- les profondeurs prédéterminées ;
- un taux de variation dans le temps de la longueur d'onde du signal lumineux cohérent.

4. Système OCT (**100**) selon l'une quelconque des revendications précédentes, dans lequel :
- chaque valeur singulière correspondant à un échantillon de signal électrique respectif, à l'exception de la valeur singulière correspondant au premier échantillon de signal électrique de la séquence, n'est pas supérieure à la valeur singulière correspondant à l'échantillon de signal électrique précédent dans la séquence,
- l'unité de traitement (**125**) est configurée pour sélectionner ledit sous-ensemble des échantillons de signal électrique reçus en sélectionnant des échantillons de signal électrique correspondant à des valeurs singulières supérieures à un seuil.

5. Système OCT (**100**) selon la revendication 4, dans lequel ledit seuil est égal à une fraction de la valeur singulière la plus élevée parmi les valeurs singulières pouvant être obtenues par la décomposition de ladite matrice de coefficients.

6. Système OCT (**100**) selon l'une quelconque des revendications précédentes, dans lequel l'unité de traitement (**125**) est configurée pour sélectionner ledit sous-ensemble des échantillons de signal électrique reçus en sélectionnant, parmi tous les échantillons de signal électrique de la séquence, une sous-séquence comprenant des échantillons de signal électrique initiaux dans la séquence et en rejetant des échantillons de signal électrique ultérieurs dans la séquence.

7. Système OCT (**100**) selon l'une quelconque des revendications précédentes, dans lequel ledit sous-ensemble des échantillons de signal électrique reçus comprend un nombre des échantillons de signal électrique reçus, ledit nombre dépendant de la différence entre la longueur d'onde la plus élevée et la longueur d'onde inférieure du signal lumineux cohérent pendant ledit intervalle de temps.

8. Système OCT (**100**) selon l'une quelconque des revendications précédentes, dans lequel les variables inconnues dudit système d'équations linéaires comprennent les valeurs de réflectivité de l'ensemble multipliées par un terme dépendant de valeurs de phase desdits échantillons de signal électrique, lesdites valeurs de phase étant des fonctions desdites profondeurs prédéterminées.

9. Système OCT (**100**) selon l'une quelconque des revendications précédentes, dans lequel l'unité de traitement est configurée pour générer un A-scan de l'objet cible (**O**) sur la base de l'ensemble des valeurs de réflectivité.

10. Système OCT (**100**) selon l'une quelconque des revendications précédentes, dans lequel :
- l'objet cible (**O**) est une zone rétinienne d'un œil, et
- la longueur d'onde de la lumière cohérente balaie, pendant ledit intervalle de temps, un intervalle de balayage en longueur d'onde comprenant une longueur d'onde correspondant à 1064 nm.

11. Système OCT (**100**) selon la revendication 10, dans lequel :
- la différence entre la longueur d'onde la plus élevée et la longueur d'onde inférieure de la lumière cohérente dans ledit intervalle de balayage en longueur d'onde correspond à 11 nm, et l'unité de traitement (**125**) est configurée pour sélectionner ledit sous-ensemble des échantillons de signal électrique reçus en sélectionnant des échantillons courants correspondant à des valeurs singulières supérieures à un seuil inférieur à 90, de préférence correspondant à 40, ou
- la différence entre la longueur d'onde la plus élevée et la longueur d'onde inférieure de la lumière cohérente dans ledit intervalle de balayage en longueur d'onde correspond à 22 nm, et l'unité de traitement (**125**) est configurée pour sélectionner ledit sous-ensemble des échantillons de signal électrique reçus en sélectionnant des échantillons courants correspondant à des valeurs singulières supérieures à un seuil inférieur à 65, de préférence correspondant à 20, ou
- la différence entre la longueur d'onde la plus élevée et la longueur d'onde inférieure de la lumière cohérente dans ledit intervalle de balayage en longueur d'onde correspond à 44 nm, et l'unité de traitement (**125**) est configurée pour sélectionner ledit sous-ensemble des échantillons de signal électrique reçus en sélectionnant des échantillons courants correspondant à des valeurs singulières supérieures à un seuil inférieur à 45, de préférence correspondant à 5, ou
- la différence entre la longueur d'onde la plus élevée et la longueur d'onde inférieure de la lumière cohérente dans ledit intervalle de balayage en longueur d'onde correspond à 110 nm, et l'unité de traitement (**125**) est configurée pour sélectionner ledit sous-ensemble des échantillons de signal électrique reçus en sélectionnant des échantillons courants correspondant à des valeurs singulières supérieures à un seuil inférieur à 40, de préférence correspondant à 31.

12. Méthode pour réaliser une tomographie par cohérence optique (OCT) sur un objet cible (**O**), la méthode comprenant:
- générer un signal lumineux cohérent ayant une longueur d'onde qui varie dans le temps sur un intervalle de temps ;
- diviser le signal lumineux cohérent en un signal aller de référence dirigé vers un réflecteur de référence (**R**), et en un signal aller opératif dirigé vers l'objet cible (**O**) ;
- détecter un signal lumineux d'interférence généré par une combinaison d'un signal retour de référence revenant du réflecteur de référence (**R**) et d'un signal retour opératif revenant de l'objet (**O**) ;
- générer un signal électrique d'interférence détecté indicatif du signal lumineux d'interférence, dans lequel ladite génération d'un signal électrique d'interférence détecté comprend :
- recevoir une séquence d'échantillons de signal électrique du signal électrique d'interférence détecté collectés à des instants d'échantillonnage correspondants à l'intérieur de l'intervalle de temps ;
- traiter les échantillons de signal électrique reçus pour calculer, pour chaque profondeur prédéterminée parmi un ensemble de profondeurs prédéterminées à l'intérieur de l'objet cible, une valeur de réflectivité correspondante indicative de la réflectivité de l'objet cible à ladite profondeur prédéterminée, de manière à obtenir un ensemble correspondant de valeurs de réflectivité, dans lequel ledit traitement des échantillons de signal électrique reçus comprend :
- calculer chaque valeur de réflectivité sous la forme d'une sommation correspondante d'échantillons de signal électrique, chacun étant pondéré par un poids respectif, dans lequel lesdits poids correspondent à des valeurs singulières pouvant être obtenues par la décomposition d'une matrice de coefficients d'un système d'équations, chaque équation du système exprimant une relation entre l'un des échantillons de signal électrique reçus et au moins l'une des valeurs de réflectivité de l'ensemble, et dans lequel ladite sommation implique un sous-ensemble des échantillons de signal électrique reçus, la méthode comprenant en outre :
- générer un vecteur de réflectivité dans lequel chaque élément du vecteur de réflectivité est une valeur correspondante parmi lesdites valeurs de réflectivité de l'ensemble de valeurs de réflectivité ; et
- générer un A-scan de l'objet cible sur la base des éléments dudit vecteur de réflectivité.
